Europäisches Patentamt

European Patent Office

Office européen des brevets

(19)

(11) Publication number: 0 205 352

A2

(12) **EUROPEAN PATENT APPLICATION**

(21) Application number: 86304535.7

(22) Date of filing: 12.06.86

(51) Int. Cl.⁴: C 12 P 21/00
G 01 N 33/577

(30) Priority: 12.06.85 JP 127844/85

(43) Date of publication of application:
17.12.86 Bulletin 86/51

(84) Designated Contracting States:
BE CH DE FR IT LI NL SE

(71) Applicant: SHIONOGI & CO., LTD.
12, Dosho-machi 3-chome Higashi-ku
Osaka 541(JP)

(72) Inventor: Mikawa, Haruki
6-16, Oharanokamizatokatsuyama-cho
Nishigyo-ku Kyoto-shi Kyoto(JP)

(72) Inventor: Hosoi, Susumu Kotohaitsufushimiinari C-525
1-1, Fukakusashimogawara-cho Fushima-ku
Kyoto-shi Kyoto(JP)

(72) Inventor: Miyake, Tamotsu
10-17, Nanguu-cho
Ashiya-shi Hyoto(JP)

(72) Inventor: Harada, Shigenori
1-6-2-601, Takanodai
Suita-shi Osaka(JP)

(74) Representative: Bizley, Richard Edward et al,
BOULT, WADE & TENNANT 27 Furnival Street
London EC4A 1PQ(GB)

(54) Monoclonal anti-human IgG4 antibodies, their production and use.

(57) A monoclonal anti-human $IgG_4$ antibody is provided, which antibody may be produced by a hybridoma of an antibody forming cell and a myeloma mouse cells, which shows a high afinity for human $IgG_4$, particularly for specific human $IgG_4$ antibody bound to antigens, but not for other human IgG subclasses, and which recognizes the Fc fragment of human $IgG_4$ and belongs to mouse isotype $IgG_2b$.

EP 0 205 352 A2

### MONOCLONAL ANTI-HUMAN IgG$_4$ ANTIBODIES, THEIR PRODUCTION AND USE

This invention relates to monoclonal anti-human IgG$_4$ antibodies, their production and use.

In allergic diseases due to antigen-antibody reactions it has become possible to effect diagnosis and classification of the condition of such diseases and the selection of an adequate choice of therapeutic course thanks to detection and identification of the causative substances (allergens) being enabled. This invention provides monoclonal antibodies useful as diagnostic agents for identifying allergens in allergic diseases such as exogenous bronchial asthma, atopic dermatitis, immune complex diseases etc. and for elucidation of their onset-mechanism. They can also be used for quantification of human serum total IgG$_4$ and the purification of polyclonal human IgG$_4$.

Of the four kinds of IgG subclasses, human IgG$_4$ is the minimal component (1 - 4% of the total IgG) and its blood level is usually 0.2 - 1 mg/ml.

W.E. Parish reported that IgG$_4$ has activity as an antibody causing anaphylaxis following a short time after sensitizing (Lancet, 2, 591 (1970)), while van der Giessen et al. states that IgG$_4$ also has another activity as a blocking antibody to inhibit IgE-mediated histamine release from mast and basophilic cells (Int. Arch. Allergy Appl. Immunol., 50, 625 (1976)). Thus, many researchers have come to be interested in the role which human IgG antibody plays in immediate allergin diseases such as bronchial asthma and atopic dermatitis. Recently,

0205352

demand for a quantitative assay for human serum allergen-specific $IgG_4$ antibody has increased.

When a conventional polyclonal anti-human $IgG_4$ antibody is used, its cross-reactive components are absorbed and removed by human $IgG_1$, $IgG_2$ and $IgG_3$ so that its affinity for human $IgG_4$ is elevated. This decreases its potency and makes it difficult to prepare anti-human $IgG_4$ antibody for practical use. On the other hand, such attempts as have been made using monoclonal anti-human $IgG_4$ antibody prepared by a hybridoma method (J. Lowe et al., Immunol., 47, 329 (1982) and R. Djurup et al., Allergy, 39, 51 (1984)) resulted in an evaluation of the specificity of the antibody to IgG subclasses which was not satisfactory because such attempts usually used IgG subclasses derived from human myeloma serum not polyclonal human IgG subclasses, i.e. antibody specificity was not sufficiently guaranteed since reactions with myeloma serum $IgG_1$, $IgG_2$, or $IgG_3$ partly occurred upon repeated evaluation.

The development of a new assay for human allergen specific $IgG_4$ antibody whose human IgG subclass specificity is guaranteed is strongly desired. A monoclonal antibody of the present invention can be produced in large quantities in the abdominal cavity of a mouse as an antibody whose properties are always homogenous. Accordingly, it is made possible to assay allergen specific-human $IgG_4$ antibody by preparing a monoclonal anti-human $IgG_4$ antibody which can bind to a common and unique epitope of human $IgG_4$ with an equaly affinity.

The monoclonal anti-human $IgG_4$ antibody (e.g.

antibody HG4-53G) of this invention, which may be produced by a hybridoma of an antibody forming cell and a mouse myeloma cell, has the following characteristics:-

1) The antibody shows a high affinity for human $IgG_4$ but does not bind to other human IgG subclasses.

2) It shows high affinity for a specific human $IgG_4$ antibody bound to antigens, but does not bind to other specific human IgG subclass antibodies.

3) It recognizes the Fc fragment of human $IgG_4$.

4) It is a mouse $IgG_2b$.

Antibody having the above properties can be used for assay of allergen-specific $IgG_4$ antibody and immune complex by RAST or ELISA and in the elucidation of the onset mechanism of allergic diseases. Since the antibody shows little non-specific affinity in normal serum, it can also be used for assay of allergen-specific $IgG_4$ antibody by making use of commercial allergen disks prepared for assay of IgE antibody and carriers immobilizing allergens (for example, coated tubes, beads, etc.).

In the drawings:-

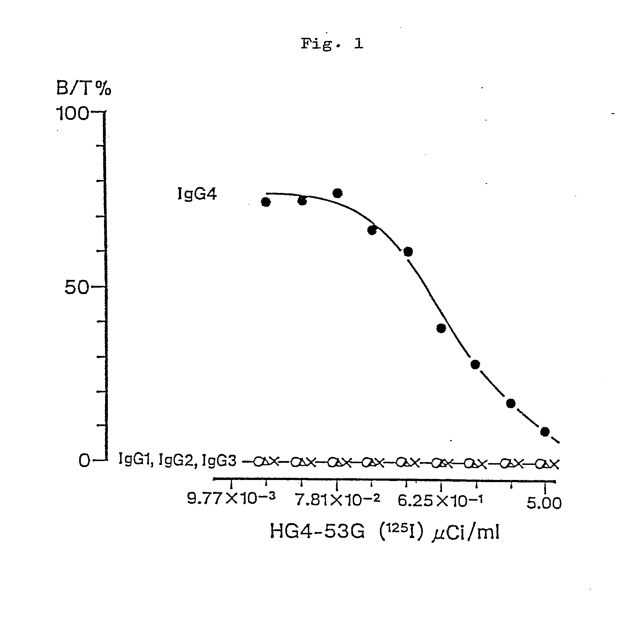

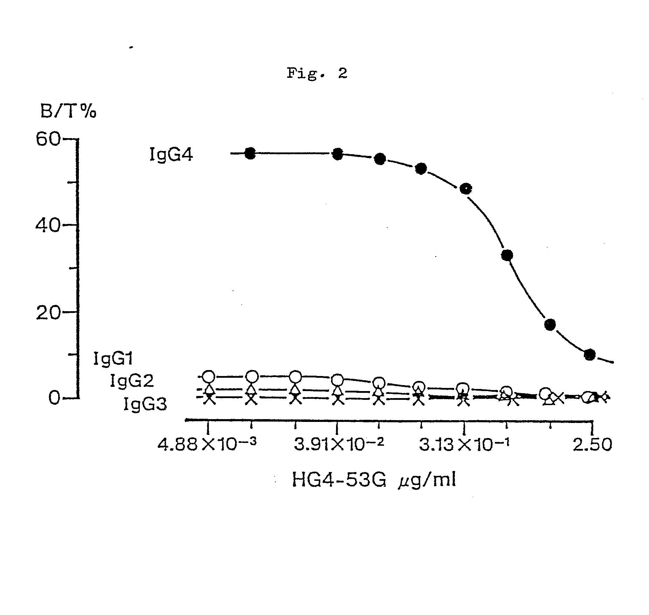

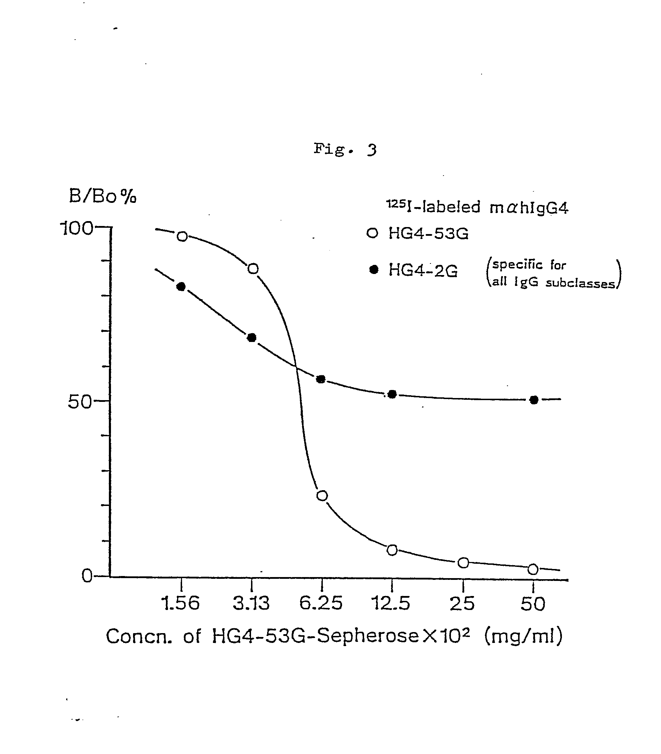

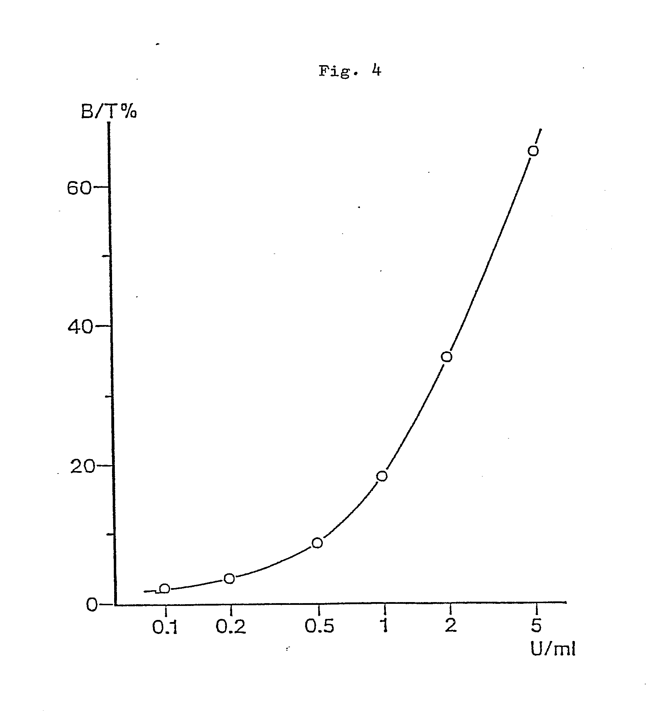

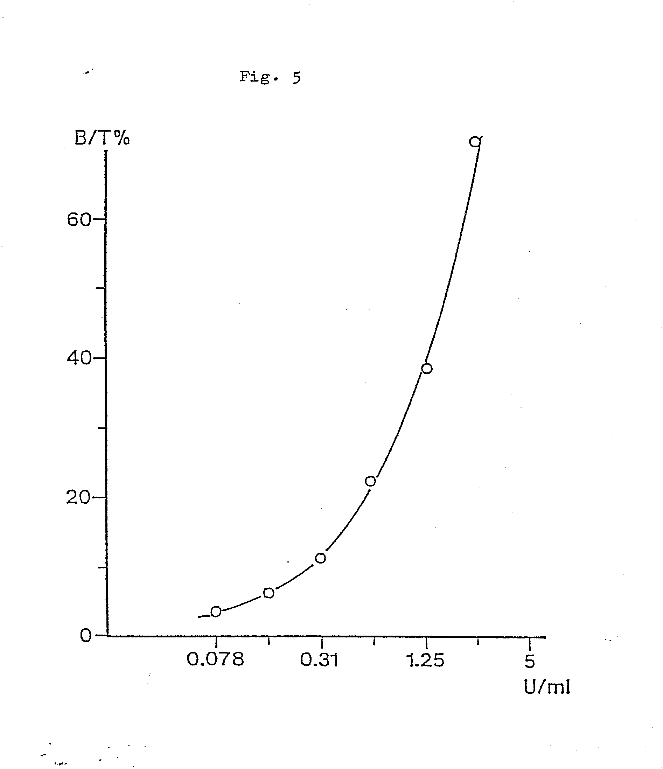

Fig. 1 shows the affinity of $^{125}$I-labeled HG4-53G for each subclass of IgG non-specifically bound to a microtiter plate, with the concentration of $^{125}$I-labeled HG4-53G added for reaction as the abscissa and the percentage of $^{125}$I-labeled HG4-53G binding to each human IgG subclass as the ordinate. Fig. 2 shows the affinity of $^{125}$I-labeled HG4-53G for each human IgG subclass in solution, with the concentration of HG4-53G added for reaction as the abscissa and the percentage of $^{125}$I-labeled HG4-53G binding to each human IgG subclass as the ordinate. Fig. 3 shows the influence in the egg-albumin specific IgG assay system, when $IgG_4$ in the samples was absorbed with immobilized anti-himan $IgG_4$ antibody (HG4-53G), with the concentration of immobilized anti-human $IgG_4$ used for absorption and removal of $IgG_4$ as the abscissa and the % between the amount (Bo) of $^{125}$I-labeled HG4-53G or $^{125}$I-labeled HG4-53G binding to the disk without absorption of $IgG_4$ and that (B) of their binding to the disk with absorption of $IgG_4$, as the ordinate. Fig. 4 shows a standard curve of $^{125}$I-labeled HG4-53G used in the Phadebas IgG RAST system against bee vemon, with the concentrations of bee venom-specific IgG standard solution as the abscissa and % of $^{125}$I-labeled HG4-53G binding to the disk as the ordinate. Fig. 5 shows a standard curve of $IgG_4$ RAST against an egg-albumin allergen disk, with the concentrations of egg-albumin specific $IgG_4$ as the abscissa and % of $^{125}$I-labeled HG4-53G binding to the disk as the ordinate.

## Preparation of monoclonal antibody against human immunoglobulin (IgG, etc.)

(1) Preparation of antibody-producing spleen cells

To animals, such as mice, rats etc. immunoglobulin derived from human serum was intraperitoneally administered together with Freund's complete adjuvant for immunization. Furthermore, about 3 weeks later, immunoglobulin derived from human serum (an additional stimulating amount) was intraperitoneally given together with alum adjuvant. Three days later the spleen was isolated to prepare a cell suspension fluid and use it as an antibody-producing cell for fusion.

(2) Preparation of myeloma cell

A hypoxanthine-guanine-phosphoribosyl transferase deficiency myeloma cell strain, for example, myeloma cell P3.NS-1/1.Ag4.1 of BALB/C mouse (hereafter abbreviated as NS-1/Ag) (Proc. Natl. Acad. Sci., U.S.A., 76, 4061 (1979)), was maintained in a medium, for example, RPMI-1640 medium containing 15% fetal calf serum (FCS). Growing of NS-1/Ag was inhibited in hypoxanthine aminopterin thymidine medium (HAT).

(3) Preparation of hybridoma

The spleen cells prepared above (1) and the myeloma cells NS-1/Ag prepared above (2) were fused in the presence of polyethylene glycol (PEG) according to the method of Oi and Herzenberg (Selected Methods in Cellular Immunology, 351 - 372 (1980)), San Francisco, W.H. Freeman and Co.). Hybrid cells thus obtained were suspended in HAT medium and the suspension inoculated in wells on tissue culture plates. It was desirable that a small amount of thymus cell or the peritoneal exudate of BALB/C strain mouse exists therein as a nutrient cell. On days, 1, 2, 3, 5, 8 and 11 after initiation of culture, half the amount of HAT medium of each well was exchanged for new HAT medium and, furthermore, on days 14, 15 and 16 the half amount was exchanged with a medium containing hypoxanthine thymidine alone (HT medium).

(4)    Assay of anti-human immunoglobulin antibody producing hybridoma

(A)    Assay based upon passive hemagglutination using human immunoglobulin-sensitized sheep erythrocytes

According to the chromium chloride method (E.R. Gold and H.H. Fudenberg, J. Immunol., 99, 859 (1967)), sheep erythrocytes washed with chromium trichloride were sensitized with human immunoglobulin.  Similarly, sheep erythrocytes sensitized with FC fragment, F (a, b')$_2$ or human myeloma protein can be obtained.  Using the sensitized sheep erythrocytes, the antibody-producing potency in culture supernatant was assayed by passive erythrocyte hemagglutination.

(B)    Assay using ELISA method

Human immunoglobulin was immobilized on the wells of polystyrene microtiterplate, using 0.05M carbonate buffer (pH 9.1).  After the remaining binding sites were saturated with bovine serum albumin, the immunoglobulin was reacted with the culture-supernatant of the hybridoma.  After washing, it was reacted with peroxidase-conjugated anti-mouse immunoglobulin antibody having no cross-affinity for human immunoglobulin.  After washing, the resultant was reacted with a substrate (hydrogen peroxide and ABTS) and the absorbance of the reactant measured after stopping the reaction with sodium azide.

(5)    Cloning and selection of hybridomas

Hybridomas whose antibody activity has been recognized in the above procedures (14) were further cloned using a limiting dilution method well known in this technical field. The culture supernatant of the thus-grown clones was further assayed for antibody activity in accordance with the methods (4) to select clones with high antibody activity.

(6) Preparation of anti-human immunoglobulin (IgG, etc.) monoclonal antibody

The clones selected in the above (5) were cultured in a suitable medium, for example, RPMI-1640 medium containing 15% of FCS, until the cell density reached its upper limit. Alternatively, the selected clones were transplanted into the abdominal cavity of BALB/C strain mouse etc. treated with pristane and ascites retained collected about 3 weeks later.

(7) Separation and purification of monoclonal antibody

Separation and purification of the monoclonal antibody from the culture-supernatant after cultivation of the ascites, from which the cells are removed by centrifugation, was easily carried out according to methods well known in this technical field, such as affinity chromatography using protein A Sepharose.

As shown in Experimental examples described later, when monoclonal anti-human $IgG_4$ antibody of the present invention was assayed for its affinity for human IgG subclasses by the ELISA method, it showed a high affinity for human $IgG_4$, but not for human $IgG_1$, $IgG_2$ and $IgG_3$. In addition, it

bound to the Fc fragment of $IgG_4$, but not to the Fab fragment. Its isotype was proved to be $IgG_2b$ by an immunoprecipitation method.

Examples

(1) Preparation of anti-human $IgG_4$ antibody-producing hybridoma

BALB/C strain mice (8 - 10 weeks of age) were immunized by intraperitoneal administration of 150 - 200 $\mu g$ (100 $\mu l$ physiological saline) of $IgG_4$ derived from human serum together with 100 $\mu l$ of Freund's complete adjuvant. Twenty one days later, 20 $\mu g$ of $IgG_4$ derived from human serum was, furthermore, intraperitoneally administered as alum sediment to each mouse. Three days later, the spleen was isolated and a cell suspension fluid was prepared using RPMI-1640 medium, and 10 ml of RPMI-1640 medium containing 5 x 10⁸ of spleen cells was mixed with 5 x 10⁷ of NS-1 myeloma cells previously suspended in 10 ml of RPMI-1640 medium. This mixture was centrifuged at 500 x g for 10 minutes to remove the supernatant. Next, 1 ml of RPMI-1640 medium containing 50% PEG4000 (Merck Co.,) was added to the mixture with gently stirring. Moreover, after stirring for 1 minute, an additional amount of 1 ml of RPMI-1640 medium was added in one minute. After another 1 ml of the same medium was added, 7 ml of the same medium was further added in about 3 minutes, then the mixture being centrifuged at 500 x g for 10 minutes to remove the supernatant. The cells obtained were suspended in 20 ml of RPMI-1640 medium containing 15% fetal calf serum (hereafter on abbreviated as FCS), 100 $\mu l$ each of which was inoculated to each well of 2 sheets of tissue culture plates (96-well, Cohning Co.,) to be cultured at 37°C under the atmosphere of 7% carbon dioxide gas.

On days 1, 2, 3, 5 and 8 thereafter, half amount of each medium was exchanged with HAT medium (RPMI-1640 medium supplemented with 10% FCS, $4 \times 10^{-7}M$ aminopterin, $1.6 \times 10^{-5}M$ thymidine and $1 \times 10^{-4}M$ hypoxanthine) and then cultured further. On days 11, 13 and 14 half amount of the medium was exchanged with HT medium (RPMI-1640 medium supplemented with 10% FCS, $1.6 \times 10^{-5}M$ thymidine and $1 \times 10^{-4}M$ hypoxanthine).

Sixteen days after initiation of fusion procedures, colonies of hybridomas appeared in an average of 85% of the wells. These hybridomas were cultured in RPMI-1640 medium containing 15% FCS to examine the existence of specific antibody production as follows:

The culture-supernatant was used as a fluid to be examined, 50 $\mu$ l of which was taken out to be added to the wells where human IgG₄ was immobilized, and react at 37°C for one hour. After washing, 50 $\mu$ l of peroxidase-conjugated anti-mouse IgG antibody was added to react at 25°C for one hour. And then after washing, 50 $\mu$ l of a substrate solution ($H_2O_2$ + ABTS) was added thereto. The reaction was stopped 5 -30 minutes later by adding 50 $\mu$ l of 2mM-NaN₃ and the absorbance of the reactant was measured at 450nm.

The results are shown in table 1.

Table 1

| Experimental No. | No. of whole wells | No. of colony appearing wells | No. of antibody producing wells |
|---|---|---|---|
| 1 | 384 | 384 | 113 |
| 2 | 384 | 384 | 233 |
| 3 | 576 | 320 | 240 |
| 4 | 576 | 576 | 440 |

(2) Cloning of anti-human IgG₄ antibody producing cell strains

Anti-human IgG₄ antibody producing hybridomas obtained in (1) were suspended to make a concentration of 5 hybridomas/ml in RPMI-1640 medium containing 15% FCS and then the suspension was inoculated into 40 wells of a tissue culture plate (96-well, Cohning Co.,) to make each well to 100 $\mu$ l. Next, the said suspension was diluted to a concentration of one hybridoma/ml to be inoculated into 32 wells, and then further diluted to a concentration of 0.5 hybridoma/ml to be inoculated into other 24 wells. Five days later 100 ml of 15% FCS + RPMI-1640 medium was added to each well. In the case of clone HG4-53G, hybridoma clone appeared from the well of at the concentration of 0.5 hybridoma/ml. The antibody activity against human IgG subclasses was

-11-

assayed on the culture-supernatant of the clone by ELISA method.

Experimental results

Antibody activity against human $IgG_4$ could be detected but not against $IgG_1$, $IgG_2$ and $IgG_3$.

(3) Production of anti-human $IgG_4$ antibody

The anti-human $IgG_4$ antibody-producing hybridoma ($10^7$ cells) obtained in the previous procedures was suspended in 0.5 ml of physiological saline, and then the suspension was transplanted into the abdominal cavity of BALB/C mouse treated with 0.5 ml of pristane in advance. Three weeks later an abdominal hyperplasia was confirmed and the ascites taken out with a syringe and centrifuged in order to remove the cells.

(4) Separation and purification of anti-human $IgG_4$ antibody

The ascites collected in the preceding procedure were salted out with 18% sodium sulfate, and a resulting precipitation was solved in 0.01M borate buffered saline (pH 8.0), which was dialyzed with the same saline. 20 mg of $\gamma$-fraction obtained by salting-out was dissolved in 2 ml of 0.01M borate buffered saline (pH 8.0), and the solution was adsorbed into a column (1.6 x 5 cm) of protein A-Sepharose (Pharmacia AB). At first, impurities were eluted with about 50 ml of 0.01M borate buffered saline (pH 8.0), and then with about 100 ml of 0.01M citrate buffered saline (pH 3.5) to obtain monoclonal anti-human $IgG_4$ antibody HG4-53G.

Experimental example 1

Iodine-labeling of monoclonal antibody HG4-53G (IODO-GEN method)

Fifty $\mu$l of dichloromethane solution containing 1, 3, 4, 6-tetrachloro-3$\alpha$,6$\alpha$-diphenyl glycoluryl (purchased as IODO-GEN from Pierce Chemical Co.,) at the concentration of 40 $\mu$g/ml was put into a glass tube and was subjected to air-drying at a room temperature in a stream of nitrogen. To this tube HG4-53G antibody solution (30 $\mu$g/15 $\mu$l) dissolved in 15 $\mu$l of 0.1M phosphate buffer (pH 7.4) was added and 0.5 mCi/5 $\mu$l of radioactive sodium iodide (Na$^{125}$I) was also added to shake gently at a room temperature for 15 minutes. The reactant solution obtained was passed through a column (1.6 x 50 cm) of Sephadex G-25 (Pharmacia AB) and was eluted with phosphate buffered saline. The first eluted fraction was separated to obtain $^{125}$I-labeled monoclonal antibody HG4-53G.

Experimental example 2

Affinity of monoclonal antibody HG4-53G for human IgG subclasses

(1) Affinity for IgG subclasses nonspecifically bound with microtiter plate

Into each well of polystyrene-made microtiter plate (Immulon 2 plate, Dynatech Co.) 50 $\mu$l of 0.25M carbonate buffer (pH 9.1) containing 0.5 $\mu$g/ml of human IgG subclass (IgG$_1$, IgG$_2$, IgG$_3$ or IgG$_4$) was placed. This plate was allowed to stand at 37°C for 2 hours and then washed with 0.1 M phosphate buffer (pH 7.4) (buffer A) containing 0.1% serum albumin, 0.05% Tween 20 and 0.01% sodium azide, and then it was further allowed to stand in buffer A at 37°C for one hour. Next, after washing twice with buffer A, 50 $\mu$l of $^{125}$I-labeled HG4-53G solution (0.020 - 5 $\mu$Ci/ml) was added to each well. This plate was maintained at a room temperature for 16 hours and washed twice with buffer A. The amount of $^{125}$I-labeled HG4-53G antibody bound to human IgG subclasses of each well was determined by $^{125}$I radioassay.

Experimental results

The binding % (B/T%) of $^{125}$I-labeled HG4-53G against each subclass of human IgG nonspecifically bound to the wells is shown in Fig. 1. HG4-53G had an affinity for human $IgG_4$, but not for other human IgG subclasses ($IgG_1$, $IgG_2$ and $IgG_3$) at all. The affinity constant for human $IgG_4$ was calculated according to Scatchard's method and it was $K = 2.57 \times 10^{10}$ 1/mol.

(2) Affinity for human IgG subclasses in a solution state

$^{125}$I-labeled HG4-53G was diluted to 0.5 $\mu$Ci/ml with 0.01M phosphate buffered saline (pH 7.4) (buffer B) containing 0.1% bovine serum albumin. To 50 $\mu$l of this solution and 50 $\mu$l of HG4-53G solution at the concentration of 0.005 - 2.5 $\mu$g/ml (HG4-53G dissolved in buffer B), 0.1 ml of buffer B was added, and then 50 $\mu$l of 2.5 $\mu$g/ml human IgG subclass ($IgG_1$, $IgG_2$, $IgG_3$ or $IgG_4$) solution was also added thereto to react at 25°C for 16 hours.

Then, mouse monoclonal antibody with a high affinity for human $\kappa$ chain and $\lambda$ chain each was mixed with the said solution. 5 mg of the mixture was added to 1 ml of cyan bromide-activated Sepharose carrier (Pharmacia AB) to react, resulting in an immobilized anti-human ($\kappa$ + $\lambda$) antibody. To each test tube 0.1 ml of this immobilized 2nd antibody was added to react at 25°C for one hour with stirring. After washing the antibody 3 times with 2 ml of buffer B, $^{125}$I

-14-

radioactivity of each test tube was determined.

Experimental results

The binding % (B/T%) of $^{125}I$-labeled HG4-53G to each subclass of human IgG existing in a solution state is shown in Fig. 2. The labeled substance showed a high affinity for human $IgG_4$, but scarcely for other human IgG subclasses ($IgG_1$, $IgG_2$ and $IgG_3$). Calculation of the affinity constant according to Scatchard's method revealed that $K = 4.72 \times 10^{10}$ 1/mol.

(3) Affinity for human IgG subclasses having specifically bound to allergen disk

Five mg of monoclonal antibody HG4-53G, which had been confirmed to bind strongly to human $IgG_4$, but not to other human IgG subclasses, was added to 1 ml of cyan bromide activated Sepharose carrier (Pharmacia AB) to react each other to obtain an anti-human $IgG_4$ antibody immobilized. Fifty-fold diluted solution of egg-albumen specific IgG rich serum was mixed with equivalent volume of 0.015 -0.5 mg/ml immobilized anti-human $IgG_4$ antibody to react at 4°C for 16 hours and then centrifuged. The supernatant (50 $\mu$1) was made to react with each of RAST egg-albumen allergen disks (Phadebas RAST, Pharmacia AB) at 25°C for 3 hours. After washing, 50 $\mu$1 each of $^{125}I$-labeled HG4-2G, a monoclonal antibody which binds to all kinds of human IgG subclasses, and $^{125}I$-labeled HG4-53G, each at 2.2 $\mu$Ci/ml, was added to react at 25°C for 16 hours. After washing, $^{125}I$ radioactivity was determined

Experimental results

As shown in Fig. 3, even if a high level of solid phase anti-human $IgG_4$ antibody was made to react in order to remove $IgG_4$ in the sample, $^{125}$I-labeled HG4-2G showed an affinity since other specific human IgG subclasses ($IgG_1$, $IgG_2$ and $IgG_3$) remained therein, but $^{125}$I-labeled HG4-53G did not bind to other subclasses except specific $IgG_4$ since it did not react with those other subclasses.

Experimental example 3

(1) Assay of bee venom-specific $IgG_4$ antibody

To bee venom allergen disk of IgG RAST (Phadebas IgG RAST, Pharmacia AB), 50 $\mu$l of a standard solution containing 5 U/ml - 0.1 U/ml bee venom-specific IgG antibody was added to react at 25°C for 3 hours. After washing, 50 $\mu$l of $^{125}$I-labeled HG4-53G (2.2 $\mu$Ci/ml) was added to react at 25°C for 16 hours, and $^{125}$I radioactivity was measured after washing.

Experimental results

A standard curve of bee venom-specific $IgG_4$ is shown in Fig. 4, indicating a high B/T% proportional to the concentrations of bee venom-specific $IgG_4$ and the background values due to non-specific IgG were also extremely low.

(2) Assay of egg-albumen specific $IgG_4$ antibody

To RAST egg-albumen allergen disk (Phadebas RAST, Pharmacia AB) 50 $\mu$l of 200 - 12,800 fold diluted solution of egg-albumen-specific $IgG_4$ rich serum was added to react at 25°C for 3 hours. After washing, 50 $\mu$l of $^{125}$I-labeled HG4-53G (2.2 $\mu$Ci/ml) was added to react at 25°C for 16 hours and $^{125}$I radioactivity was determined after washing.

## Experimental results

The dilution curve of egg-albumen-specific $IgG_4$ is shown in Fig. 5, indicating high binding ratio B/T% in proportion to the concentration of egg-albumen-specific $IgG_4$, and the background levels due to non-specific IgG were also remarkably low.

CLAIMS:

1.    A monoclonal antibody which belongs to the mouse $IgG_2b$ isotype, which recognizes the Fc fragment of human $IgG_4$, and which has an affinity for human $IgG_4$, particularly having an affinity for specific human $IgG_4$ bound to an antigen, but not for other specific human IgG subclasses.

2.    Monoclonal antibody HG4-53G.

3.    A process for the production of monoclonal antibodies, which process comprises forming hybridomas between antibody-producing cells of an animal immunized by human $IgG_4$ and myeloma cells, selecting hybridomas producing anti-human $IgG_4$ antibody, cloning said selected hybridomas, optionally according to a limited dilution method, to give clones which produce antibodies with an affinity for human $IgG_4$, and isolating anitbodies not having an affinity for human $IgG_1$, $IgG_2$ and $IgG_3$ but having an affinity for specific human $IgG_4$ bound to an antigen but not for other specific human IgG subclasses.

4.    A process as claimed in claim 3, wherein said isolation is carried out by transplanting said clones into the abdominal cavity of a mouse previously treated with pristane for proliferation purposes, allowing said clones to produce monoclonal antibody in the ascites and separating/purifying said antibody.

5.    A process as claimed in claim 4, wherein said purification of monoclonal antibody is carried out by affinity chromatography using a protein A-binding carrier resin.

6.    A process as claimed in claim 5, wherein said carrier resin is Sepharose.

7.    An anti-human $IgG_4$ antibody-producing hybridoma capable of producing an antibody as defined in claim 1.

8.    The use of an antibody as defined in claim 1 in: (a) diagnosis other than when practised on the human or animal body; or (b) assay of $IgG_4$; or (c) purification of polyclonal $IgG_4$; or (d) elucidation of the onset mechanism of an allergic disease.

0205352

1/5

Fig. 1

B/T%
100

IgG4

50

0 — IgG1, IgG2, IgG3 —αx—αx—αx—αx—αx—αx—αx—αx—αx

9.77×10⁻³    7.81×10⁻²    6.25×10⁻¹    5.00

HG4-53G (¹²⁵I) μCi/ml

2/5

Fig. 2

Fig. 3

B/Bo%

$^{125}$I-labeled mαhIgG4

○ HG4-53G

● HG4-2G ( specific for all IgG subclasses )

Concn. of HG4-53G-Sepherose×10² (mg/ml)

Fig. 4

Fig. 5